# EUROPEAN PATENT APPLICATION

(11) **EP 3 574 917 A1**
(43) Date of publication of application: **04.12.2019**
(21) Application number: 18744156.3
(22) Date of filing: 09.01.2018
(51) Int. Cl.: A61K 45/00, A61K 31/711, A61K 48/00, A61P 35/00, A61P 35/02, A61P 43/00

(54) **ANTI-CANCER AGENT, METHOD FOR SCREENING FOR ANTI-CANCER AGENT, KIT FOR DETERMINATION OF EFFICACY ON CANCER, AND METHOD FOR DETERMINING EFFICACY ON CANCER**

(30) Priority: 27.01.2017 JP 2017012654
(71) Applicant: University of Miyazaki, Miyazaki-shi Miyazaki 889-2192 (JP)
(72) Inventor: MORISHITA Kazuhiro, Miyazaki-shi Miyazaki 889-1692 (JP); ICHIKAWA Tomonaga, Miyazaki-shi Miyazaki 889-1692 (JP); NAKAHATA Shingo, Miyazaki-shi Miyazaki 889-1692 (JP)
(74) Representative: v. Bezold & Partner Patentanwälte - PartG mbB
(86) International application number: PCT/JP2018/000173
(87) International publication number: WO 2018/139181

(57) **Abstract**

An anticancer agent includes a protein arginine methyltransferase 5 inhibitor, and is used against a cancer in which the expression level of NDRG2 in a cancer cell is lower than that in a corresponding normal cell. The cancer may be selected from the group consisting of adult T-cell leukemia-lymphoma, osteosarcoma, and cervical cancer.

## Description

### Technical Field

The present disclosure relates to an anticancer agent, a method of screening an anticancer agent, a kit for determining efficacy against a cancer, and a method of determining efficacy against a cancer.

### Background Art

Protein arginine N-methyltransferase 5 (PRMT5) is a protein arginine methyltransferase associated with methylation modification of an arginine residue in a protein. An arginine-methylated biological molecule regulates transcription in a nucleus, and is involved in DNA repair. Such an arginine-methylated biological molecule regulates a cell function such as a signal transduction system in cytoplasm. Arginine-methylated biological molecules are prominently involved in various cancers. Therefore, the inhibition of PRMT5 is considered to be one of the targets of cancer therapies. For example, Patent Literature 1 discloses that a PRMT5 inhibitor is efficacious against plural kinds of cancers such as leukemia.

### Citation List

### Patent Literature

Patent Literature 1: National Patent Publication No. 2016-514700
Patent Literature 2: International Publication No. WO 2010/116899

### Non Patent Literature

Non Patent Literature 1: Shingo Nakahata, and 21 others, "Loss of NDRG2 expression activates PI3K-AKT signalling via PTEN phosphorylation in ATLL and other cancers", 2014, 5: 3393, DOI: 10. 1038/ncomms 4393

### Summary of Invention

### Technical Problem

However, a PRMT5 gene encoding PRMT5 has also been reported to function as a cancer suppressor gene depending on the kind of cancer. The details of the kind of cancer of which PRMT5 involvement in the maintenance and proliferation of the cancer cells occurs to obtain the effect of a PRMT5 inhibitor have not been revealed. Known PRMT5 inhibitors include those causing function inhibitory without discrimination between normal and cancer cells. Thus, the PRMT5 inhibitors have been demanded to be improved in selectivity for cancer cells.

The present disclosure was made under such actual circumstances with an objective to provide an anticancer agent capable of offering high selectivity for a cancer cell, a method of screening an anticancer agent, a kit for determining efficacy against a cancer, and a method of determining efficacy against a cancer.

### Solution to Problem

In Non Patent Literature 1 and Patent Literature 2, the present inventors have reported that a gene, N-Myc downstream-regulated gene 2 (NDRG2), is a cancer suppressor gene for various cancers such as adult T-cell leukemia-lymphoma (ATLL), liver cancer, and lung cancer.

NDRG2 protein encoded by NDRG2 forms, together with PP2A phosphatase, a complex, and dephosphorylates a key protein group that includes phosphatase and tensin homologue deleted on chromosome 10 (PTEN) and is important for signal transduction, to change activity. In addition, the present inventors identified PRMT5 as a binding protein of which the phosphorylation modification is changed by NDRG2 protein, and intensively repeated researches to accomplish the present disclosure.

In other words, an anticancer agent according to a first aspect of the present disclosure includes:
a protein arginine methyltransferase 5 inhibitor,
the anticancer agent being used against a cancer in which an expression level of NDRG2 in a cancer cell is lower than that in a corresponding normal cell.

In such a case, the cancer may be selected from a group consisting of adult T-cell leukemia-lymphoma, osteosarcoma, and cervical cancer.

A method of screening an anticancer agent according to a second aspect of the present disclosure includes:
a contact step of bringing a protein arginine methyltransferase 5 inhibitor into contact with a cancer cell in which an expression level of NDRG2 is lower than that in a corresponding normal cell.

A kit for determining efficacy against a cancer according to a third aspect of the present disclosure
is a kit for determining efficacy of a protein arginine methyltransferase 5 inhibitor against a cancer, and
includes a reagent for quantifying an expression level of NDRG2 in a cancer cell.

A method of determining efficacy against a cancer according to a fourth aspect of the present disclosure
is a method of determining efficacy of a protein arginine methyltransferase 5 inhibitor against a cancer, and
includes:
a measurement step of measuring an expression level of NDRG2 in a cancer cell;
a comparison step of comparing the expression level measured in the measurement step with an expression level of NDRG2 in a corresponding normal cell; and
a determination step of determining that the protein arginine methyltransferase 5 inhibitor is efficacious against the cancer cell when the expression level of NDRG2 in the cancer cell is lower than the expression level of NDRG2 in the normal cell in the comparison step.

### Advantageous Effects of Invention

In accordance with the present disclosure, high selectivity for a cancer cell can be obtained.

### Brief Description of Drawings

FIG. 1 is a view illustrating the expression of NDRG2 in cell lines Jurkat, MOLT4, HUT102, KOB, U2OS, and HeLa;
FIG. 2A is a view illustrating variations in the cellular proliferative potential of cells according to HUT102 with time;
FIG. 2B is a view illustrating variations in the cellular proliferative potential of cells according to KOB with time;
FIG. 3A is a view the expression of cleaved (CL.)-caspase3 which is a marker of apoptosis in cells according to HUT102;
FIG. 3B is a view illustrating the expression of CL.-caspase3 in cells according to KOB;
FIG. 4A is a view illustrating the images of cells according to HUT102 with stained CL.-caspase3, in which the length of each bar is equivalent to 100 µm;
FIG. 4B is a view illustrating the ratios of cells with positive CL.-caspase3 to cultured cells according to HUT102;
FIG. 5A is a view illustrating the images of cell lines according to KOB with stained CL.-caspase3, in which the length of each bar is equivalent to 100 µm;
FIG. 5B is a view illustrating the ratios of cells with positive CL.-caspase3 to cultured cells according to KOB;
FIG. 6 is a view illustrating variations, with time, in the volumes of tumors in mice in which cells according to HUT102 are subcutaneously transplanted;
FIG. 7 is a view illustrating the weights of the tumors in the mice in which the cells according to HUT102 are subcutaneously transplanted;
FIG. 8 is a view illustrating the images of tumors collected 31 days after subcutaneously transplanting the cells according to HUT102;
FIG. 9 is a view illustrating the results of immunohistochemical staining of the tumors illustrated in FIG. 8, in which the length of each bar is equivalent to 200 µm;
FIG. 10A is a view illustrating variations in the cellular proliferative potential of HUT102 with time in the presence of a PRMT5 inhibitor AdOX;
FIG. 10B is a view illustrating variations in the cellular proliferative potential of KOB with time in the presence of AdOX;
FIG. 11A is a view illustrating variations in the cellular proliferative potential of Jurkat with time in the presence of AdOX;
FIG. 11B is a view illustrating variations in the cellular proliferative potential of MOLT4 with time in the presence of AdOX;
FIG. 12A is a view illustrating variations in the cellular proliferative potential of cells according to U2OS with time;
FIG. 12B is a view illustrating variations in the cellular proliferative potential of cells according to HeLa with time;
FIG. 13A is a view illustrating variations in the cellular proliferative potential of U2OS with time in the presence of AdOX; and
FIG. 13B is a view illustrating variations in the cellular proliferative potential of HeLa with time in the presence of AdOX.

### Description of Embodiments

Embodiments according to the present disclosure will be described with reference to the accompanying drawings. The present disclosure is not limited to the following embodiments and drawings.

### (Embodiment 1)

First, an anticancer agent according to Embodiment 1 will be described. The anticancer agent according to the present embodiment includes a PRMT5 inhibitor. The PRMT5 inhibitor is not particularly limited as long as the PRMT5 inhibitor directly or indirectly inhibits PRMT5. The PRMT5 inhibitor is, for example, a compound, an antibody, a protein, a peptide, a DNA aptamer, an RNA aptamer, or the like that binds to PRMT5 to suppress the activity of PRMT5. The PRMT5 inhibitor may inhibit PRMT5 through suppression of the expression of a PRMT5 gene. In this case, the PRMT5 inhibitor is an siRNA, an antisense nucleic acid, or the like.

Preferably, the PRMT5 inhibitor is a compound. Preferred examples of the compound as the PRMT5 inhibitor include AdOX (adenosine-2',3'-dialdehyde), EPZ0 15666, AMI-1,5'-methylthioadenosine, and S-adenosylhomocysteine.

The anticancer agent described above is used against a cancer in which the expression level of NDRG2 in a cancer cell is lower than that in a corresponding normal cell. A standard method known in the art can be used for measuring the expression of NDRG2. For example, the expression level of NDRG2 can be measured based on, as an index, the amount of mRNA into which NDRG2 is transcribed, or the amount of NDRG2 protein. The amounts of mRNA and NDRG2 protein can be measured using a standard method based on the base sequence of NDRG2 and the amino acid sequence of the NDRG2 protein, of which each is known.

In the case of measuring the amount of mRNA which is a transcription product of NDRG2 in a cancer cell, quantification of the mRNA may be performed by electrophoresis after PCR of cDNA into which the mRNA is reversely transcribed, using a specific primer, or by a real-time PCR method using a fluorescently labeled probe.

In the case of measuring the amount of NDRG2 protein in a cancer cell, for example, the amount of the protein can be measured by using an antibody binding to the protein. The antibody may be a polyclonal antibody or a monoclonal antibody. The quantification of the protein may be performed by immunohistochemical staining using the antibody, or by using an immunoblot method or the like, in which the quantification is performed using an antibody after electrophoresis of a protein extract obtained from a cancer cell.

"Corresponding normal cell" means a cell that is not cancerated in contrast to a cancerated cell. A normal cell as a comparison target may be a normal cell from a subject, or may be a normal cell from an individual different from the subject. The average value of the measured expression levels of NDRG2 in normal cells from plural individuals may be regarded as a comparison target. Further, a proper value such as average value ± standard deviation may also be regarded as a comparison target on the basis of the data of the expression levels in plural normal cells, measured in advance.

Preferably, the anticancer agent described above is used against a cancer in which the expression level of NDRG2 in a cancer cell is significantly lower than that in a corresponding normal cell. Herein, "significantly lower" means that a level is lower by a statistically significant difference. For example, the difference between the average expression level of cancer cells and the average expression level of normal cells may be subjected to a test such as t-test in order to evaluate a statistical difference.

For example, the corresponding normal cell is a T cell, a cell that is not cancerated in a lymph node, or the like in a case in which the cancer is ATLL. The corresponding normal cell is, for example, a cell that is not cancerated in a site corresponding to the site of origin of a tumor in a case in which the cancer is osteosarcoma. The corresponding normal cell is, for example, a cell that is not cancerated in the uterine cervix in a case in which the cancer is cervical cancer.

More specifically, the expression level of NDRG2 in the cancer cell described above is 0 to 90%, 0 to 80%, 0 to 70%, 0 to 60%, 0 to 50%, 0 to 40%, 0 to 30%, 0 to 20%, 0 to 10%, 0 to 5%, 0 to 3%, 0 to 2%, or 0 to 1% as a relative value with respect to the expression level of NDRG2 in the corresponding normal cell.

Preferred examples of the cancer in which the expression level of NDRG2 in a cancer cell is lower than that in the corresponding normal cell include ATLL, pancreatic cancer, skin cancer, malignant lymphoma, prostatic cancer, endometrial cancer, cervical cancer, ovarian cancer, head and neck squamous cancer, esophageal cancer, bile duct cancer, prostatic cancer, pheochromocytoma, penile cancer, osteosarcoma, and testicular cancer. Such cancer cells in which the expression levels of NDRG2 are lower are described in Non Patent Literature 1 and Patent Literature 2.

The anticancer agent according to the present embodiment is produced to include a PRMT5 inhibitor as an active constituent by a known method. The anticancer agent includes 0.1 to 99% by weight, 1 to 50% by weight, and preferably 1 to 20% by weight of the PRMT5 inhibitor as the active constituent.

The dosage form of the anticancer agent according to the present embodiment is preferably an injectable agent or an oral agent. In addition, the dosage form of the anticancer agent may be a rectal suppository, a vaginal suppository, a pernasal absorbent, a transdermal absorbent, a transpulmonary absorbent, an intraoral absorbent, or the like. The anticancer agent may be a mixture in which a pharmaceutically acceptable carrier is blended. Examples of the pharmaceutically acceptable carrier include various organic carrier substances or inorganic carrier substances. The pharmaceutically acceptable carrier is blended, in the anticancer agent, as, for example, an excipient, a lubricant, a binder, or a disintegrant in a solid preparation, or a solvent, a solubilizing agent, a suspending agent, an isotonizing agent, a buffer agent, or a soothing agent in a liquid preparation. In addition, an additive such as an antiseptic agent, an antioxidant, a colorant, or a sweetener can also be used as needed.

The excipient is, for example, lactose, sucrose, D-mannitol, starch, crystalline cellulose, light anhydrous silicic acid, or the like. The lubricant is, for example, magnesium stearate, calcium stearate, talc, colloidal silica, or the like. The binder is, for example, crystalline cellulose, sucrose, D-mannitol, dextrin, hydroxypropyl cellulose, hydroxypropyl methylcellulose, polyvinyl pyrrolidone, or the like. The disintegrant is, for example, starch, carboxymethyl cellulose, carboxy methylcellulose calcium, croscarmellose sodium, sodium carboxymethyl starch, or the like.

The solvent is, for example, water for injection, alcohol, propylene glycol, macrogol, or the like. The solubilizing agent is, for example, polyethylene glycol, propylene glycol, D-mannitol, benzyl benzoate, ethanol, tris-aminomethane, cholesterol, triethanolamine, sodium carbonate, sodium citrate, or the like. The suspending agent is a surfactant, a hydrophilic macromolecule, or the like, and is, for example, stearyl triethanolamine, sodium lauryl sulfate, laurylaminopropionic acid, lecithin, benzalkonium chloride, benzethonium chloride, glyceryl monostearate, polyvinyl alcohol, polyvinyl pyrrolidone, sodium carboxymethylcellulose, methyl cellulose, hydroxymethyl cellulose, hydroxyethyl cellulose, hydroxypropyl cellulose, or the like.

The isotonizing agent is, for example, sodium chloride, glycerin, D-mannitol, or the like. The buffer agent is, for example, a buffer of a phosphate, an acetate, a carbonate, or a citrate, or the like. The soothing agent is, for example, benzyl alcohol or the like. The antiseptic agent is, for example, a para-hydroxybenzoate ester, chlorobutanol, benzyl alcohol, phenethyl alcohol, dehydroacetic acid, sorbic acid, or the like. The antioxidant is, for example, a sulfite, ascorbic acid, or the like.

The dose of the anticancer agent according to the present embodiment is determined by, for example, the gender, age, body weight, and symptom of a patient, as appropriate. The anticancer agent is administered so that the amount of PRMT5 inhibitor is a therapeutically effective amount. The effective amount is the amount of PRMT5 inhibitor, required for obtaining a desired result, and is an amount required for causing the delay, inhibition, prevention, reversal, or cure of the progress of a condition subjected to therapy or treatment. The dose of the anticancer agent is typically 0.01 to 1000 mg/kg, preferably 0.1 to 200 mg/kg, and more preferably 0.2 to 20 mg/kg. The anticancer agent may be administered once a day, or more than once a day in divided doses. The anticancer agent may be administered at various dosing frequencies, such as every day, every other day, once a week, every other week, and once a month. The anticancer agent may be used in an amount outside the ranges described above, as needed.

A method of administering the anticancer agent according to the present embodiment is not particularly limited, and the anticancer agent is administered through an injection, a pernasal, transdermal, transpulmonary, or oral route, or the like. Intravenous administration, intraarterial administration, or oral administration is particularly preferred as the administration method.

The anticancer agent according to the present embodiment hardly influences the cell proliferation of cancer cells in which the expression level of NDRG2 is not lower, while exhibiting suppression of the cell proliferation of cancer cells in which the expression level of NDRG2 is lower, as illustrated in the following Example 4. Since the expression level of NDRG2 is not lower in a normal cell that is not cancerated, high selectivity for a cancer cell can be obtained. The anticancer agent suppresses cell proliferation specific for a cancer cell, and therefore enables a reduction in the risk of a side effect.

The anticancer agent is preferably used against a cancer selected from the group consisting of ATLL, osteosarcoma, and cervical cancer. Anticancer activities against the cancer cells of ATLL, osteosarcoma, and cervical cancer can be more reliably obtained as described in the following Examples 2 to 4.

### (Embodiment 2)

A method of screening an anticancer agent according to Embodiment 2 will now be described. The method of screening an anticancer agent according to the present embodiment includes a contact step of bringing a PRMT5 inhibitor into contact with a cancer cell in which the expression level of NDRG2 is lower than that in a corresponding normal.

The cancer cell in which the expression level of NDRG2 is lower is, for example, a cell collected from the tumor tissue of a patient, or a cancerated cell line established from the cell. The lower expression level of NDRG2 can be evaluated by the method described above. For example, the PRMT5 inhibitor may be added to a culture medium of the cancer cell in order to bring the PRMT5 inhibitor into contact with the cancer cell.

The PRMT5 inhibitor promising as an anticancer agent can be selected by evaluating the behavior of the cancer cell after the contact step. In the evaluation of the behavior of the cancer cell, a cancer cell cultured in the absence of the PRMT5 inhibitor, and a cancer cell with which the PRMT5 inhibitor is brought into contact may be compared with each other. The behavior of the cancer cell is, for example, cell proliferation, cell death, apoptosis, a change in cell cycle, or the like.

Preferably, in the contact step, cancer cells are cultured as control in a culture medium to which the PRMT5 inhibitor is not added, and the numbers of cancer cells cultured in the presence of the PRMT5 inhibitor and the control cancer cells after a lapse of a predetermined period of time may be compared. The PRMT5 inhibitor may be selected as an anticancer agent in a case in which the number of the cancer cells cultured in the presence of the PRMT5 inhibitor is significantly less than that of the control.

For example, the expression of a marker for apoptosis, such as CL.-caspase3, in the cancer cell may be evaluated by Western blot technique, immunostaining, or the like in the case of evaluating apoptosis. The PRMT5 inhibitor allowing a significantly large amount of a marker for apoptosis to be expressed than that in the control may be selected as an anticancer agent.

As described in detail above, a PRMT5 inhibitor exhibiting drug efficacy against a specific cancer can be more reliably selected with high selectivity in accordance with the method of screening an anticancer agent according to the present embodiment.

### (Embodiment 3)

Subsequently, a kit for determining efficacy against a cancer according to Embodiment 3 will be described. The kit for determining efficacy is a kit for determining the efficacy of a PRMT5 inhibitor against a cancer, and includes a reagent for quantifying the expression level of NDRG2 in a cancer cell.

The cancer cell is, for example, a cancer cell collected from the tumor tissue of a patient in a biopsy. The cancer cell may be used on an as-is basis, or cancer cells proliferated by culturing the cancer cell may be used. A cancer cell included in an immobilized tissue made into a section may be used as the cancer cell.

The reagent described above is, for example, an antibody for the NDRG2 protein described above, and a primer specific for mRNA which is a transcription product of NDRG2. The kit for determining efficacy may include another reagent for immunohistochemical staining, a fluorescently labeled probe, or the like, as needed.

In accordance with the kit for determining efficacy against a cancer according to the present embodiment, the expression level of NDRG2 in a cancer cell can be quantified, and therefore, the efficacy of a PRMT5 inhibitor against a cancer can be accurately determined.

The PRMT5 inhibitor is efficacious against a cancer in which the expression level of NDRG2 in a cancer cell is lower, and therefore, it is possible to use the kit for determining efficacy to quantify the expression level of NDRG2 in a cancer cell of a patient and to compare the expression level with the expression level of NDRG2 in a corresponding normal cell or the like. As a result, it can be determined whether or not the patient is a subject for administration, and therefore, the kit for determining efficacy is clinically useful.

In another embodiment, there is provided a method of determining the efficacy of a PRMT5 inhibitor against a cancer. The method of determining efficacy includes a measurement step, a comparison step, and a determination step. In the measurement step, the expression level of NDRG2 in a cancer cell is measured. In the comparison step, the expression level measured in the measurement step is compared with the expression level of NDRG2 in a corresponding normal cell. In the determination step, it is determined that the PRMT5 inhibitor is efficacious against a cancer cell in a case in which the expression level of NDRG2 in the cancer cell is lower than the expression level of NDRG2 in the normal cell in the comparison step.

In another embodiment, there is provided a cancer therapy method further including an administration step of administering a PRMT5 inhibitor to a cancer patient with cancer cells, in addition to the measurement step, comparison step, and determination step described above.

In another embodiment, there is provided a method of therapy or prevention of a cancer, including an administration step of administering a PRMT5 inhibitor to a patient with a cancer in which the expression level of NDRG2 in a cancer cell is lower than that in a corresponding normal cell. In another embodiment, a PRMT5 inhibitor is used in therapy or prevention of a cancer in which the expression level of NDRG2 in a cancer cell is lower than that in a corresponding normal cell.

### Examples

The present disclosure will be further specifically described with reference to the following Examples. However, the present disclosure is not limited to Examples.

### (Cell Line)

In the following Examples, HTLV-1-uninfected T-cell lymphocytic acute leukemia T-ALL (Jurkat and MOLT4), an HTLV-1-infected cell line (HUT102), an ATLL cell line (KOB), an osteosarcoma cell line (U2OS), and a uterine cancer cell line (HeLa) were used as cell lines. The cell lines described above were cultured in a RPM11640 or DMEM culture solution, to which 10% fetal bovine serum (FBS) supplemented or unsupplemented with IL2 (50 U/mL) was added, under 5% carbon dioxide at 37°C.

### (Antibody)

In Western blot technique and immunostaining in the following Examples, goat anti-PRMT5 (C-20) polyclonal antibody (sc-22132) or goat anti-NDRG2 (E-20) polyclonal antibody (sc-sc-19468) (manufactured by Santa Cruz Biotechnology, Inc.); mouse anti-β-actin (AC -74) monoclonal antibody (A5316) (manufactured by Sigma-Aldrich Co. LLC); or rabbit anti-Caspase3 (8G10) monoclonal antibody (# 9665) or rabbit anti-CL.-Caspase3 (Asp175) polyclonal antibody (# 9661) (manufactured by Cell Signaling Technology, Inc.) was used as a primary antibody. Horseradish peroxidase (HRP)-labeled anti-goat IgG antibody (P0449), HRP-labeled anti-mouse IgG antibody (P0260), or HRP-labeled anti-rabbit IgG antibody (P0399) (manufactured by Dako Cytomation) was used as a secondary antibody.

### (Example 1: Examination of Expression Levels of NDRG2 in Cell Lines)

The expression levels of the NDRG2 protein were confirmed, by Western blot technique, as the expression levels of NDRG2 in the Jurkat, MOLT4, HUT102, KOB, U2OS, and HeLa described above. In the Western blot technique, cultured cells were harvested, and then washed with phosphate buffered saline (PBS) (10 mM phosphate buffer, 120 mM NaCl, 2.7 mM KCl, pH 7.5). Then, 1% NP-40 buffer (1% Nonidet P-40, 50 mM Tris-HCl (pH 8.0), 150 mM NaCl, 5 mM EDTA (pH 8.0)) was added to lyse the cells. After centrifugation, an SDS sample buffer (100 mM Tris-HCl (pH 6.8), 4% SDS, 50% glycerol, 0.01% bromophenol blue, 5% β-mercaptoethanol) was added to 10 to 20 g of cell lysate, and the resultant was boiled at 95°C for 5 minutes. The obtained sample was subjected to 8% SDS-polyacrylamide electrophoresis (SDS-PAGE).

In SDS-PAGE, electrophoresis was performed in Mini-PROTEAN 3 Cell (manufactured by Bio-Rad Laboratories, Inc.) at a constant voltage of 100 V for 1 hour. A gel was transferred to a PVDF membrane (manufactured by Millipore) by Mini Trans-Blot Cell (manufactured by Bio-Rad Laboratories, Inc.) at a constant voltage of 100 V for 2 hours, and the PVDF membrane was subjected to blocking reaction with 1% bovine serum albumin (BSA) or 5% skim milk-supplemented phosphate buffered saline/Tween 20 (PBST) at a constant temperature for 1 hour. After the blocking, the PVDF membrane was subjected to reaction using the primary antibody 1000-fold diluted with Can Get signal (trade mark) Solution 1 (manufactured by TOYOBO CO., LTD.) or 5% skim milk overnight at 4°C.

The PVDF membrane was washed with PBST three times, and the PVDF membrane was subjected to reaction using the secondary antibody 2000-fold diluted with Can Get signal (trade mark) Solution 2 (manufactured by TOYOBO CO., LTD.) or 5% skim milk at room temperature for 1 hour. The PVDF membrane was washed with PBST three times, then subjected to chemiluminescence with Lumi light PLUS Western Blotting Kit (manufactured by Roche Applied Science), and subjected to image analysis with Luminescent Image Analyzer System LAS-3000 (manufactured by FUJIFILM Corporation).

### (Results)

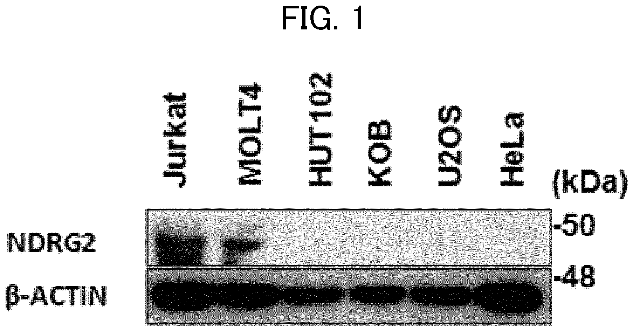

As illustrated in FIG. 1, the expression of the NDRG2 protein was observed in the cell lines Jurkat and MOLT4 uninfected with HTLV-1. In contrast, the NDRG2 protein was hardly expressed in HUT102, KOB, U2OS, and HeLa. Non Patent Literature 1 describes that the expression levels of mRNA in NDRG2 in Jurkat and MOLT4 are comparable to that in a CD4-positive T-cell.

### (Example 2: Induction of Apoptosis Due to Decreased Expression of PRMT5 Gene)

In order to decrease the expression of a PRMT5 gene in a cell line, sh-PRMT5-3 and sh-PRMT5-4 were produced as vectors for decreasing the expression of PRMT5 using knockout (trade mark) RNAi system (manufactured by Clontech Laboratories, Inc.). The base sequences of the sense and antisense strands of sh-PRMT5-3 are represented in SEQ ID NO: 1 and SEQ ID NO:2, respectively. The base sequences of the sense and antisense strands of sh-PRMT5-4 are represented in SEQ ID NO:3 and SEQ ID NO:4, respectively. Mock that did not include the base sequence of a target gene or shluc targeting luciferase was produced as a control shRNA vector.

PRMT5 expression-decreased cell lines were produced using the vectors described above. A retrovirus was produced by introducing 10 µg of such a vector described above and 20 µg of a pVSV-G vector (manufactured by Clontech Laboratories, Inc.) into a 293GP cell line by a calcium phosphate method. A Mock cell line, an shPRMT5-3 cell line, and an shPRMT5-4 cell line were established by infecting 1×10⁶ HUT102 cells and 1×10⁶ KOB cells with the obtained retrovirus.

For each established cell line, 2 ×10³ cells were cultured on a 96-well plate, and cell counting kit-8 (manufactured by DOJINDO CO., LTD) was added at a predetermined time. The number of cells was quantified based on an absorbance of 450 nm or 490 nm, to evaluate cellular proliferative potential.

Apoptosis was evaluated by Western blot technique similar to Example 1 and immunostaining of the cultured cells. In the immunostaining of the cultured cells, the cells were immobilized with 4% paraformaldehyde for 10 minutes, and subjected to permeabilization treatment with 0.1% NP-40. The cells were blocked at ordinary temperature for 1 hour using 2% BSA solution, and subjected to reaction at 4°C overnight using a primary antibody diluted with 2% BSA. The cells were washed with PBST three times, and subjected to reaction with a secondary antibody diluted with 2% BSA at room temperature for 1 hour. The cells were washed with PBST three times, and CL.-caspase3 was stained with DAB (manufactured by Thermo Fisher Scientific, Inc.).

### (Results)

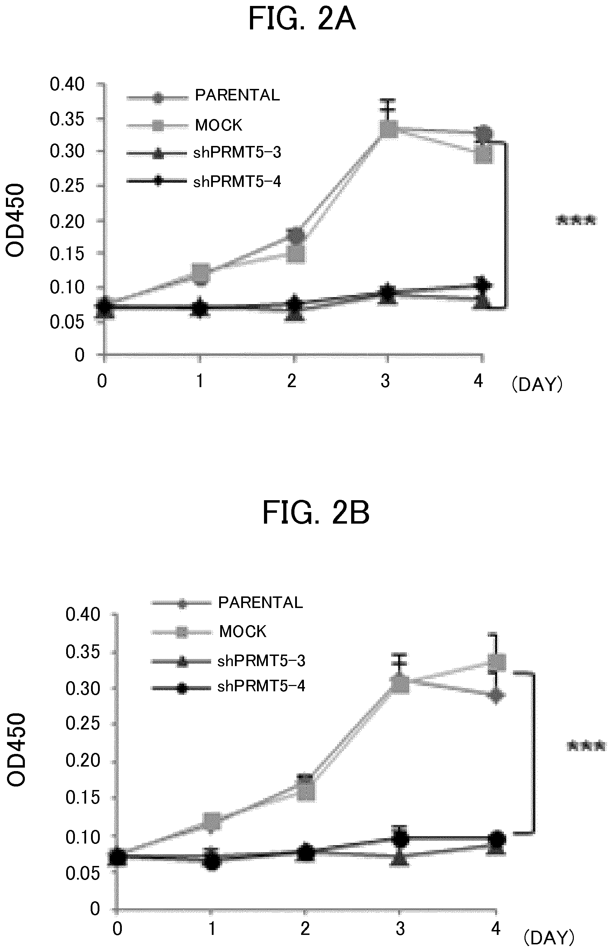

FIG. 2A illustrates variations, with time, in the cellular proliferative potential of a Mock cell line, an shPRMT5-3 cell line, and an shPRMT5-4 cell line established from HUT102 as a parent line (parental). In the shPRMT5-3 cell line and the shPRMT5-4 cell line, the cellular proliferative potential was significantly decreased due to a decrease in the expression of a PRMT5 gene. FIG. 2B illustrates variations, with time, in the cellular proliferative potential of a Mock cell line, an shPRMT5-3 cell line, and an shPRMT5-4 cell line established from KOB as a parent line. Like HUT102, the cellular proliferative potential was significantly decreased due to a decrease in the expression of a PRMT5 gene in the shPRMT5-3 cell line and the shPRMT5-4 cell line.

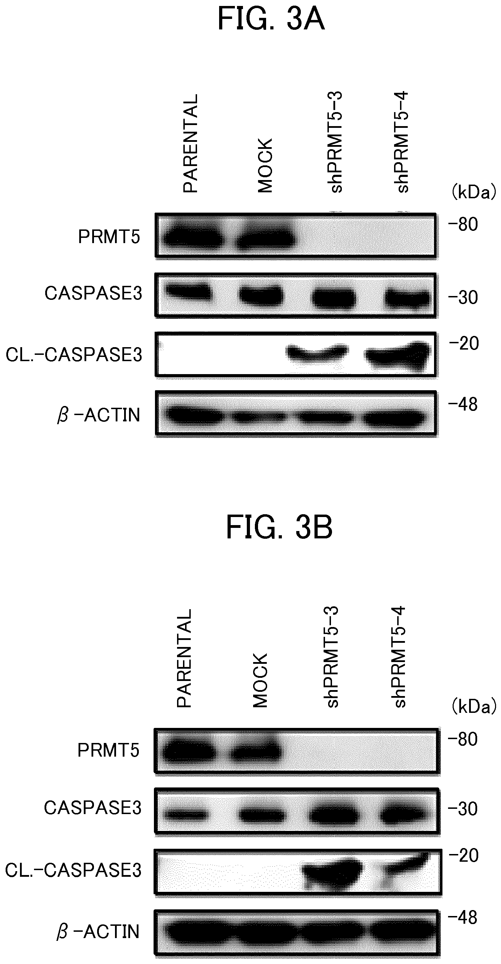

FIG. 3A and FIG. 3B illustrate the results of HUT102 and KOB subjected to Western blot technique, respectively. In both HUT102 and KOB, the expression of CL.-caspase3 as an index of apoptosis was prominently enhanced in the shPRMT5-3 cell line and the shPRMT5-4 cell line.

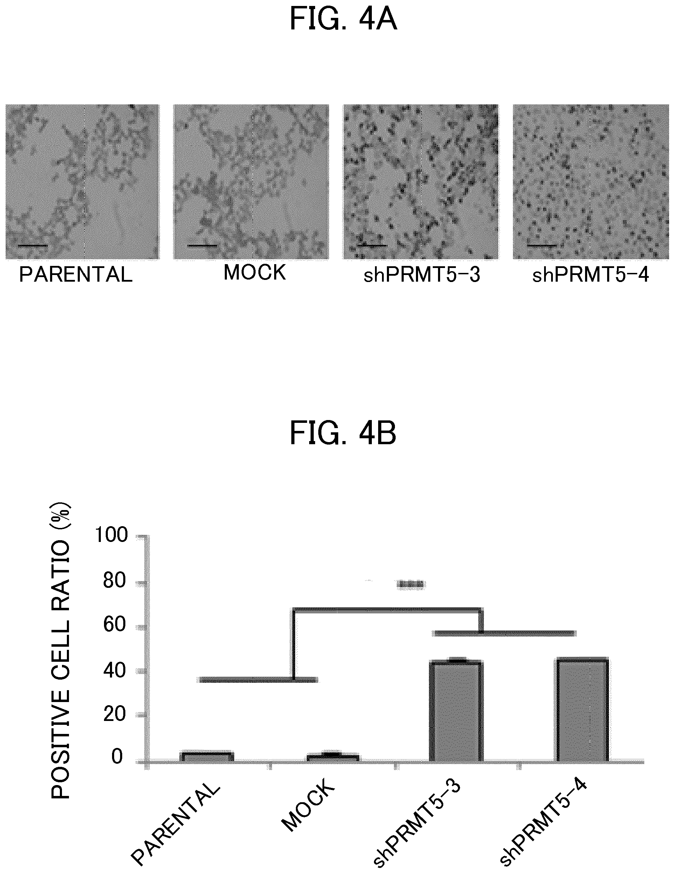

FIG. 4A illustrates the results of immunostaining of HUT102. In the immunostaining, the enhanced expression of CL.-caspase3 was also confirmed in the shPRMT5-3 cell line and shPRMT5-4 cell line of HUT102. FIG. 4B illustrates the ratios of cells with positive CL.-caspase3 in the immunostaining to the cultured cell. The significant enhancement of the expression of CL-caspase3 in the shPRMT5-3 cell line and shPRMT5-4 cell line of HUT102 was quantitatively indicated.

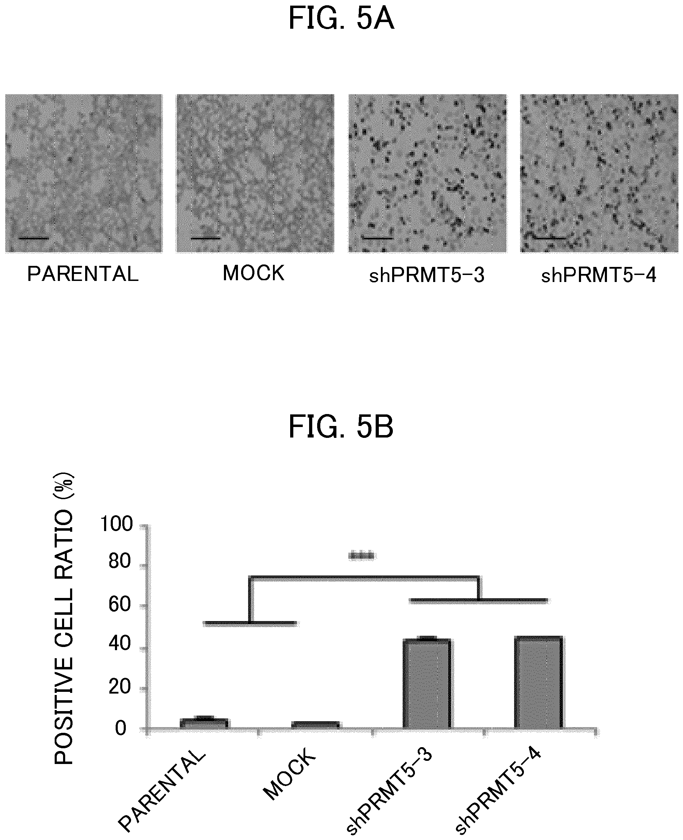

FIG. 5A and FIG. 5B illustrate the images taken in the immunostaining and the ratios of cells with positive CL.-caspase3 according to KOB, respectively. Like HUT102, the enhanced expression of CL.-caspase3 was also confirmed in the shPRMT5-3 cell line and the shPRMT5-4 cell line according to KOB.

The above results revealed that PRMT5 is involved in the cell proliferation and antiapoptosis of the cell lines according to ATLL with the decreased expression of NDRG2.

### (Example 3: Experiment on Subcutaneous Transplantation into Immunodeficient Mouse)

The parent line, Mock cell line, shPRMT5-3 cell line, and shPRMT5-4 cell line of HUT102 were harvested, 1 ×10⁷ cells of each line were suspended in PBS and Matrigel (manufactured by Becton, Dickinson and Company), and the resultant was transplanted subcutaneously into the back region of a immunodeficient SCID mouse (manufactured by CLEA Japan, Inc.) using a 26G syringe. The diameter and minor diameter of a tumor on day 7 or later after the transplantation were measured at any time by an electrically operated vernier caliper, and the volume of the tumor was calculated from a calculation expression (diameter ×(minor diameter)²/2).

The mouse was euthanized and subjected to an autopsy on day 31 after the subcutaneous transplantation, and the tumor was extracted. The weight of the tumor was measured, and the tumor was immobilized with 4% paraformaldehyde. The immobilized tumor was dehydrated with ethanol in a stepwise manner, and subjected to penetration with xylene. The tumor was embedded in paraffin. A paraffin block was thinly sliced, and immobilized on a slide glass. Paraffin was removed from the produced section using xylene, and the section was hydrophilized with ethanol in a stepwise manner. For immunohistochemical staining, an antigen was activated by microwave treatment of the section in sodium citrate (pH 6.0), followed by dipping the section in a methanol solution 0.3% H₂O₂ for 30 minutes, to remove endogenous peroxidase activity. Subsequently, the section was blocked at ordinary temperature for 1 hour using 2% BSA solution, and subjected overnight to reaction at 4°C using a primary antibody diluted with 2% BSA. Then, the section was washed with PBS three times, and allowed to react with a secondary antibody diluted with 2% BSA at room temperature for 1 hour. Further, the section was washed with PBS three times, and CL.-caspase3 was stained with DAB (manufactured by Thermo Fisher Scientific, Inc.).

### (Results)

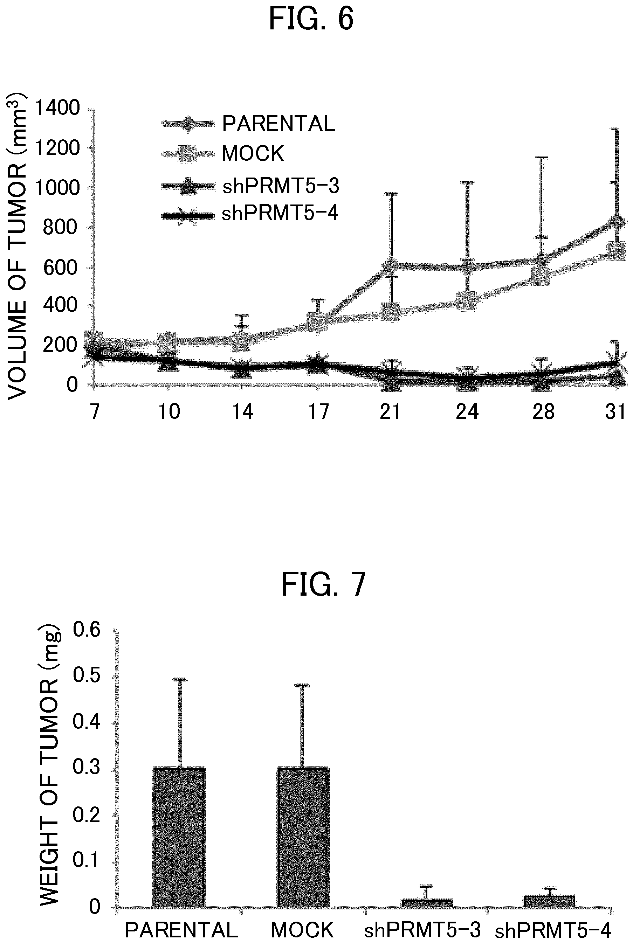

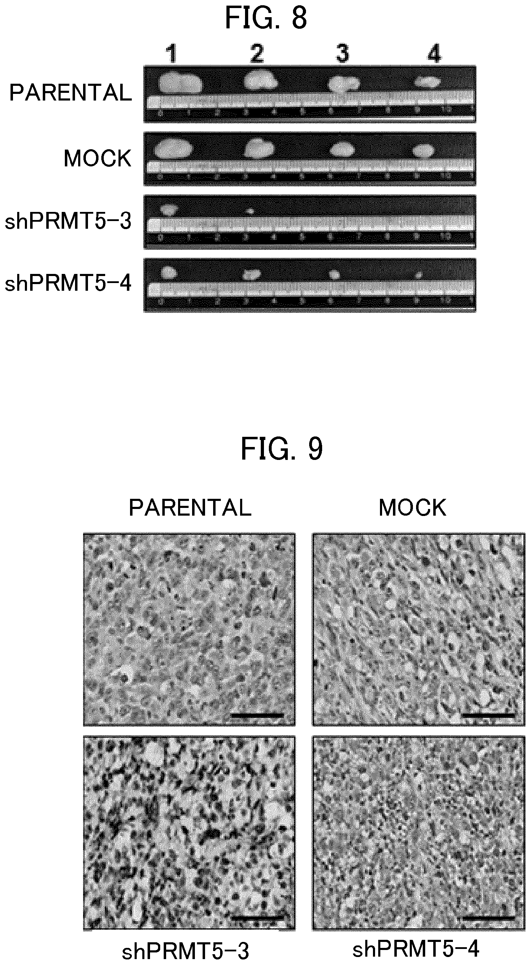

FIG. 6 illustrates variations in the volumes of the tumors with time. Tumorigenicity was significantly reduced on day 17 or later after the transplantation in the shPRMT5-3 cell line and the shPRMT5-4 cell line. As illustrated in FIG. 7 and FIG. 8, the weights and sizes of the tumors on day 31 were prominently decreased in the shPRMT5-3 cell line and the shPRMT5-4 cell line. FIG. 9 illustrates the results of immunohistochemical staining of the tumors. CL.-caspase3 was increased in the tumors of the shPRMT5-3 cell line and the shPRMT5-4 cell line.

The above results revealed that the inhibition of PRMT5 is efficacious against ATLL with the decreased expression of NDRG2 in a cancer cell.

### (Example 4: Experiment on Suppression of Cell Proliferation with PRMT Inhibitor)

In addition to HUT102 and KOB, Jurkat and MOLT4 were cultured. At the time of the start of the culture, AdOX (manufactured by Sigma-Aldrich Co. LLC) as a PRMT5 inhibitor was added to each culture medium. Like the Examples 2 described above, cellular proliferative potential was evaluated.

In the cell lines of a solid cancer, the influence of the decreased expression of a PRMT5 gene was also evaluated in a manner similar to the manner of Example 2. The cellular proliferative potential of the parent lines, shluc cell lines, shPRMT5-3 cell lines, and shPRMT5-4 cell lines of U2OS and HeLa, and an influence on the cellular proliferative potential of a PRMT inhibitor were evaluated.

### (Results)

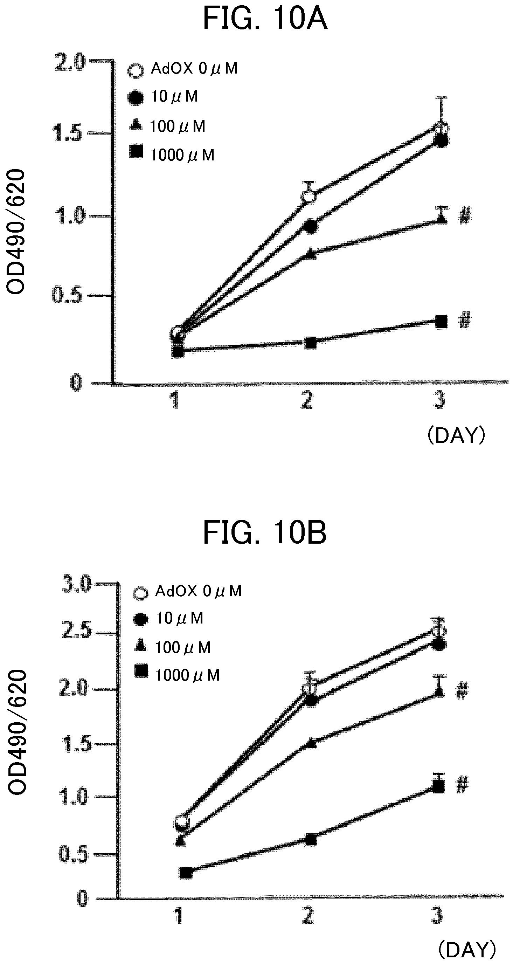

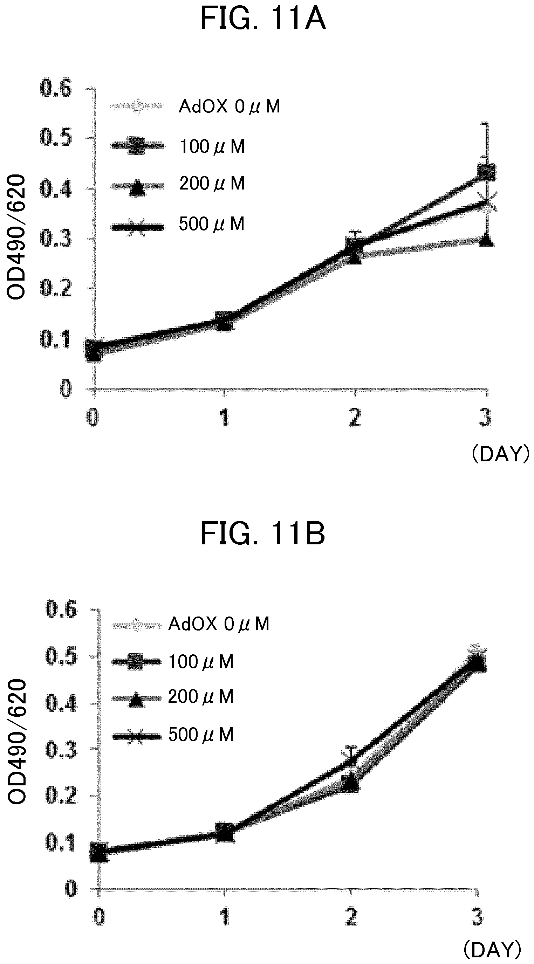

FIG. 10A and FIG. 10B illustrate variations in the cellular proliferative potential of HUT102 and KOB with time, respectively. AdOX exhibited the suppression of cell proliferation in concentration-dependent manners in both HUT102 and KOB. FIG. 11A and FIG. 11B illustrate variations in the cellular proliferative potential of Jurkat and MOLT4 with time, respectively. The suppression of cell proliferation caused by AdOX was not observed in Jurkat and MOLT4 without the decreased expression of NDRG2.

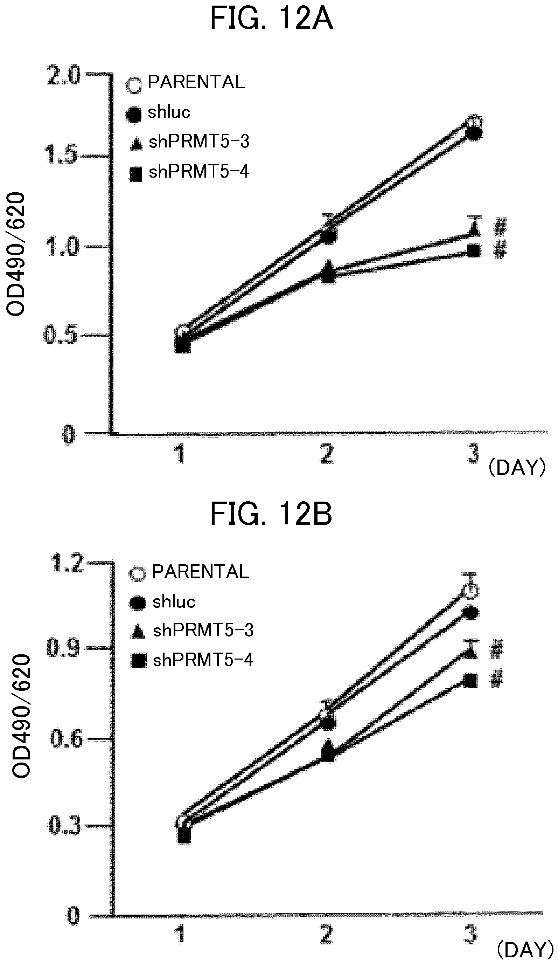

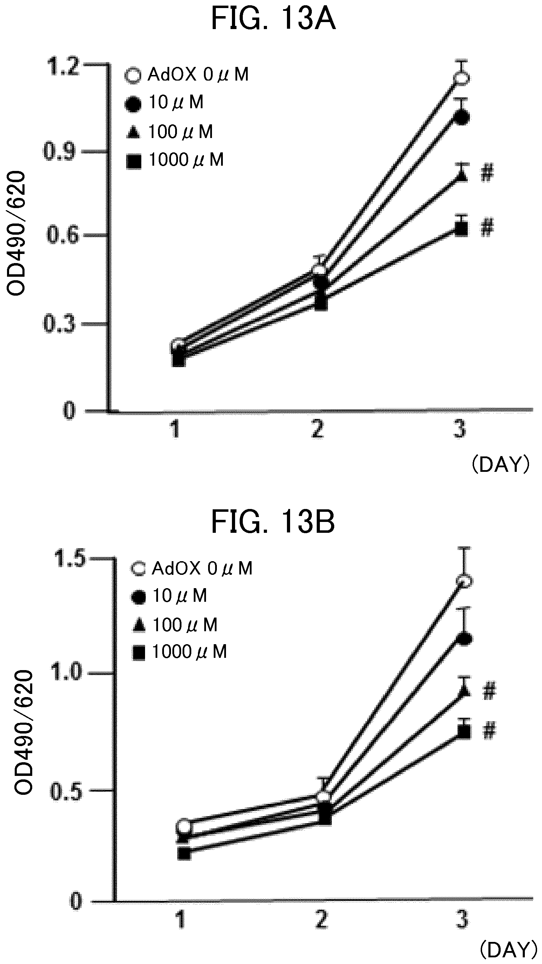

FIG. 12A and FIG. 12B illustrate variations, with time, in the cellular proliferative potential of shluc cell lines, shPRMT5-3 cell lines, and shPRMT5-4 cell lines established from U2OS and HeLa as parent lines, respectively. In the shPRMT5-3 cell lines and the shPRMT5-4 cell lines, cellular proliferative potential was significantly decreased due to the decreased expression of PRMT5 genes. FIG. 13A and FIG. 13B illustrate variations in the cellular proliferative potential of U2OS and HeLa in the presence of AdOX with time, respectively. In both U2OS and HeLa, AdOX suppressed cell proliferation in a concentration-dependent manner.

The above results revealed that the inhibition of PRMT5 is efficacious against not only ATLL but also the solid cancers with the decrease expression of NDRG2. Since the proliferation of the cancer cells without the decreased expression of NDRG2 was not suppressed by the inhibition of PRMT5, suppression of cell proliferation due to the inhibition of PRMT5 is considered to hardly occur in normal and cancer cells without the decreased expression of NDRG2.

The foregoing describes some example embodiments for explanatory purposes. Although the foregoing discussion has presented specific embodiments, persons skilled in the art will recognize that changes may be made in form and detail without departing from the broader spirit and scope of the invention. Accordingly, the specification and drawings are to be regarded in an illustrative rather than a restrictive sense. This detailed description, therefore, is not to be taken in a limiting sense, and the scope of the invention is defined only by the included claims, along with the full range of equivalents to which such claims are entitled.

This application claims the benefit of Japanese Patent Application No. 2017-12654, filed on January 27, 2017, the entire disclosure of which is incorporated by reference herein.

### Industrial Applicability

The present disclosure is suitable for therapy or prevention of a cancer, particularly a cancer with the decreased expression level of NDRG2, such as ATLL.

## Claims

1. An anticancer agent comprising:
a protein arginine methyltransferase 5 inhibitor, wherein
the anticancer agent is used against a cancer in which an expression level of NDRG2 in a cancer cell is lower than that in a corresponding normal cell.

2. The anticancer agent according to claim 1, wherein
the cancer is selected from a group consisting of adult T-cell leukemia-lymphoma, osteosarcoma, and cervical cancer.

3. A method of screening an anticancer agent, the method comprising:
a contact step of bringing a protein arginine methyltransferase 5 inhibitor into contact with a cancer cell in which an expression level of NDRG2 is lower than that in a corresponding normal cell.

4. A kit for determining efficacy against a cancer, wherein
the kit is a kit for determining efficacy of a protein arginine methyltransferase 5 inhibitor against a cancer, and
the kit comprises a reagent for quantifying an expression level of NDRG2 in a cancer cell.

5. A method of determining efficacy against a cancer, wherein
the method is a method for determining efficacy of a protein arginine methyltransferase 5 inhibitor against a cancer, and
the method comprises:
a measurement step of measuring an expression level of NDRG2 in a cancer cell;
a comparison step of comparing the expression level measured in the measurement step with an expression level of NDRG2 in a corresponding normal cell; and
a determination step of determining that the protein arginine methyltransferase 5 inhibitor is efficacious against the cancer cell when the expression level of NDRG2 in the cancer cell is lower than the expression level of NDRG2 in the normal cell in the comparison step.


SEQUENCE LISTING

<110>  University of Miyazaki

<120>  Anticancer drug, method of screening for anticancer drug, kit of
       determining effectively for cancer, and method of determining
       effectively for cancer

<130>  17F116-PCT

<150>  JP 2017-012654
<151>  2017-01-27

<160>  4

<170>  PatentIn version 3.5

<210>  1
<211>  65
<212>  DNA
<213>  Artificial Sequence

<220>
<223>  sh-PRMT5-3 sence

<400>  1
gatccgaggg agttcattca ggaattcaag agattcctga atgaactccc tcttttttac       60

gcgtg                                                                  65


<210>  2
<211>  65
<212>  DNA
<213>  Artificial Sequence

<220>
<223>  sh-PRMT5-3 antisence

<400>  2
aattcacgcg taaaaaagag ggagttcatt caggaatctc ttgaattcct gaatgaactc       60

cctcg                                                                  65


<210>  3
<211>  65
<212>  DNA
<213>  Artificial Sequence

<220>
<223>  sh-PRMT5-4 sence

<400>  3
gatccggcca tctataaatg tctgttcaag agacagacat ttatagatgg cctttttac       60

gcgtg                                                                  65


<210>  4
<211>  65
<212>  DNA
<213>  Artificial Sequence

<220>
<223>  sh-PRMT5-4 antisence

<400>  4
aattcacgcg taaaaaaggc catctataaa tgtctgtctc ttgaacagac atttatagat        60

ggccg                                                                    65